# EUROPEAN PATENT APPLICATION

(11) **EP 4 458 422 A1**
(43) Date of publication of application: **06.11.2024**
(21) Application number: 22916104.7
(22) Date of filing: 27.12.2022
(51) Int. Cl.: A61Q 19/00, A61K 8/04, A61K 8/34, A61K 8/44, A61K 8/64, A61K 8/67

(54) **SKIN CARE COMPOSITION FOR APPLICATION TO SKIN**

(30) Priority: 28.12.2021 JP 2021214170
(71) Applicant: Cosmed Pharmaceutical Co., Ltd., Kyoto-shi, Kyoto 601-8014 (JP)
(72) Inventor: QUAN, Ying-shu, Kyoto-shi, Kyoto 601-8014 (JP); TANAKA, Hiroshi, Kyoto-shi, Kyoto 601-8014 (JP); MURAYAMA, Tomohiro, Kyoto-shi, Kyoto 601-8014 (JP); KAMIYAMA, Fumio, Kyoto-shi, Kyoto 601-8014 (JP)
(74) Representative: Ter Meer Steinmeister & Partner
(86) International application number: PCT/JP2022/048211
(87) International publication number: WO 2023/127882

(57) **Abstract**

Provided are a means for crystallizing a valuable substance that is unstable in an aqueous solution state and stably dispersing the substance in an oily medium, an aqueous medium, or an emulsified medium in which the oily medium and the aqueous medium are mixed, and to a composition for application to skin with which a crystallized valuable substance is absorbed through the skin. A skin care composition for application to skin, including a valuable substance that is dispersed in a crystalline state, and a method for producing a skin care composition for application to skin, the method including a step of preparing a solution of a valuable substance at a supersaturation concentration, adding the solution to a composition for application to skin, and precipitating crystals while stirring under cooling.

## Description

### TECHNICAL FIELD

The present invention relates to a composition for application to skin characterized by causing the skin to feel a physical feel by a crystalline valuable substance, that is, a prickly feel.

### BACKGROUND ART

Hitherto, water-soluble amino acids or vitamins have been blended in compositions for application to skin in the form of being dissolved in water, and then used. However, since substances such as calcium pantothenate and ascorbic acid are oxidatively decomposed in the form of an aqueous solution, it is difficult to stably blend such substances. For this reason, various methods for stabilizing a component poor in stability have been studied. For example, Patent Document 1 describes that when calcium pantothenate and ascorbic acid are crystallized and mixed, stability is improved as compared with a mixture with non-crystallized calcium pantothenate and ascorbic acid.

Patent Document 2 describes a method for obtaining a stable oily suspension of a water-soluble vitamin by adding a water-soluble vitamin to an edible oil such as sunflower oil, corn oil, or cottonseed oil, and pulverizing the mixture until the average particle size reaches 0.1 to 100 um. However, since the composition disclosed as an example is blended in an edible oil, the composition can be used for human foods, animal feeds, and the like, but is insufficient to be used as a cosmetic preparation as it is.

Patent Document 3 describes a lipstick composition and other compositions containing a crystalline vitamin B3 compound as examples. However, the illustrated compositions are compositions free of polar solvents such as water, ethanol, and glycols, and can be applied to only a small number of compositions among cosmetic compositions.

Patent Document 4 describes a method of preparing a microspicule (fine needle) having a sharp shape by molding a mixture containing hyaluronic acid, alkylated glycerin, and water with a mold. It is described that blending such a composition in a cosmetic makes it possible to form micropores in the stratum corneum and makes components having not been transdermally absorbed so far easy to be absorbed.

Patent Document 5 describes a cosmetic using a crosslinked gelatin having an insolubilization rate of 40 to 100% by weight as a scrubbing agent, and describes that the cosmetic is a composition having moderate hardness, having a superior keratin removal effect without damaging the skin, and also having a massage effect.

Patent Document 6 discloses a composition of a scrubbing agent composed of carnauba wax having an average particle size of 50 to 1000 um, and describes a composition for application to skin which does not give uncomfortable sense of skin irritation or foreign matter feeling at the time of use, is superior in sense of use, and also is superior in scrubbing effect.

### PRIOR ART DOCUMENTS

### PATENT DOCUMENTS

Patent Document 1: JP-A-2005-325080
Patent Document 2: JP-A-2004-500392
Patent Document 3: JP-A-2002-536391
Patent Document 4: JP-A-2021-116305
Patent Document 5: JP-A-2014-172900
Patent Document 6: JP-A-2004-189612

### SUMMARY OF THE INVENTION

### PROBLEMS TO BE SOLVED BY THE INVENTION

Hitherto, many of vitamins, amino acids, and peptides are unstable in the state of an aqueous solution, are easily decomposed due to the influence of oxidation or temperature, and they have been difficult to stably blend. Therefore, they have been used while being devised such that they can be stabilized. For example, Patent Document 1 describes that when calcium pantothenate and ascorbic acid are crystallized and mixed, stability is improved as compared with a mixture with non-crystallized calcium pantothenate. Patent Document 2 describes a method for obtaining a stable liquid preparation by adding a water-soluble vitamin to an edible oil such as sunflower oil, corn oil, or cottonseed oil. Further, Patent Document 3 describes a lipstick composition containing a crystalline vitamin B3 compound. Patent Document 4 describes a method of producing microspicule particles by using hyaluronic acid and alkylated glycerin and pouring them into a mold. However, these compositions are mixtures of powders in the absence of a polar solvent such as water, or are present in a dispersed state in an oil free of a polar solvent, and their use has been limited to use in the absence of a polar solvent such as water.

In addition, Patent Document 5 discloses a method in which a gelatin protein is crosslinked to be insolubilized, a scrubbing agent having moderate hardness and failing to damage the skin is thereby prepared, and the scrubbing agent is used. Patent Document 6 discloses a method of preparing and using carnauba wax as a scrubbing agent having an average particle size of 50 to 1000 um. However, because these scrubbing agents are insoluble, they eventually do not permeate the skin and are removed from the skin as a solid.

Challenges of the present invention are to provide a means for crystallizing a valuable substance that is unstable in an aqueous solution state and stably dispersing the substance in an oily medium, an aqueous medium, or an emulsified medium in which the oily medium and the aqueous medium are mixed, and to provide a composition for application to skin with which a crystallized valuable substance is absorbed through the skin.

### MEANS FOR SOLVING THE PROBLEMS

In order to solve the challenges described above, the present inventors have extensively conducted studies, and as a result, it has become possible to stably form a microspicule (fine needle) in a crystalline state even in the presence of a polar solvent by blending a water-soluble crystal component in an aqueous solution at a concentration equal to or higher than the saturation solubility. In addition, the microspicule (fine needle) can be blended in a polar solvent system, a non-polar solvent system, and a mixed system thereof, and it has become possible to provide a composition for application to skin having a good application feel, and thus the present invention has been accomplished.

In general, it is known that substances such as amino acids, vitamins and peptides, except for oil-soluble components, are crystallized by recrystallization. By utilizing this property, it is possible to prepare an aqueous solution having a concentration equal to or higher than the saturation solubility and precipitate crystals by leaving the aqueous solution at a low temperature. Blending a component dissolved at a concentration equal to or higher than the concentration of the saturation solubility into an aqueous phase, preparing an appropriate surfactant and an oil phase, and emulsifying them have made it possible to prepare a composition for application to skin in which the component is in a crystalline state. Furthermore, it is also possible to take out a component crystallized by recrystallization and blend the component in a composition for application to skin, and it is also possible to prepare a composition in a non-polar system by blending the component in a gel oil composition.

The crystalline valuable substance can be blended in a composition for application to skin containing no polar solvent (for example, water), and can also be blended in a composition for application to skin prepared by emulsifying a polar solvent including water with a non-polar solvent such as an oil. Furthermore, the crystalline valuable substance is characterized in that while the composition for application to skin is applied to the skin and blended with the skin, the crystalline valuable substance is gradually dissolved by moisture in the composition and moisture on the skin, and eventually the crystalline component is absorbed into the skin.

The present invention is as follows.
[1] A skin care composition for application to skin, comprising a valuable substance that is dispersed in a crystalline state.
[2] A skin care composition for application to skin, comprising a valuable substance that is dispersed in a crystalline state, wherein the valuable substance is a needlelike crystal, and a length of the crystal is 5 um or more and 3000 um or less.
[3] The skin care composition for application to skin according to [1] or [2], wherein the valuable substance is water-soluble.
[4] The skin care composition for application to skin according to any one of [1] to [3], wherein the valuable substance is an amino acid.
[5] The skin care composition for application to skin according to any one of [1] to [4], wherein the valuable substance is taurine.
[6] The skin care composition for application to skin according to any one of [1] to [5], wherein the valuable substance is taurine, and is present in both a dissolved state and a crystalline state.
[7] The skin care composition for application to skin according to any one of [1] to [3], wherein the valuable substance is a peptide.
[8] The skin care composition for application to skin according to any one of [1] to [3], wherein the valuable substance is a water-soluble vitamin.
[9] The skin care composition for application to skin according to any one of [1] to [8], wherein the blending amount of the valuable substance in the composition is an amount of 1.0 to 5.0 times the saturation solubility with respect to the moisture content in the composition.
[10] The skin care composition for application to skin according to any one of [1] to [9], wherein a medium of the composition for application to skin is an oily medium, an aqueous medium, or an emulsified medium in which an oily medium and an aqueous medium are mixed.
[11] The skin care composition for application to skin according to any one of [1] to [3], wherein the valuable substance dispersed in a crystalline state is cymen-5-ol or thymol.
[12] A method for producing a skin care composition for application to skin, the method comprising a step of preparing a solution of a valuable substance at a supersaturation concentration, adding the solution to a composition for application to skin, and precipitating crystals while stirring under cooling.
[13] A method for producing a skin care composition for application to skin, the method comprising a dispersion step of preparing part of a valuable substance as a solution having a supersaturation concentration, adding the solution to a composition for application to skin, and precipitating crystals while stirring under cooling; and a step of adding part of the valuable substance direct as crystals to the composition for application to skin and dispersing the part of the valuable substance.
[14] A method for producing a skin care composition for application to skin, the method comprising a step of adding crystals of a valuable substance to a composition for application to skin and dispersing the crystals.

### EFFECT OF THE INVENTION

According to the present invention, it is possible to prepare a composition for application to skin having microspicules (fine needles) that afford a prickly feel to the skin regardless of whether it is a polar solvent system or a non-polar solvent system. In addition, even though the crystalline component is finally present in a crystalline state in the composition for application to skin, crystals of the component are dissolved during applying the composition to the skin and blending it with the skin, so that the component is absorbed by the skin. Thus, it is possible to provide a composition for application to skin that has a good sense of use, is mild to the skin, is free of skin irritation, and has an unprecedented feel.

### BRIEF DESCRIPTION OF THE DRAWINGS

FIG. 1 is an optical micrograph (150 magnifications) of taurine crystals obtained in Example 10.
FIG. 2 is a graph showing the measurement results of Example 11 and Comparative Example 4.

### MODE FOR CARRYING OUT THE INVENTION

The skin care composition for application to skin of the present invention (hereinafter, it may be briefly written as composition for application to skin) comprises a valuable substance dispersed in a crystalline state.

Examples of the valuable substance include amino acids, peptides, and water-soluble vitamins. The valuable substance is preferably water-soluble.

The valuable substance is preferably an amino acid. Examples of the amino acid that can be used in the present invention include essential amino acids such as isoleucine, leucine, valine, histidine, lysine, methionine, tryptophan, phenylalanine, and threonine; non-essential amino acids such as asparagine, aspartic acid, alanine, arginine, cysteine, cystine, glutamine, glutamic acid, glycine, proline, serine, and tyrosine; and free amino acids which do not constitute proteins such as theanine, ornithine, citrulline, taurine, and γ-aminobutyric acid. Modified amino acids such as modified amino acids such as hydroxyproline, methylated amino acids, and acetylated amino acids can also be used.

Among such amino acids, those having a low saturation solubility of less than 1 g with respect to 100 g of water at 25°C can form crystals only in a small amount, so that they hardly afford a gritty sense of use. In addition, those having a high saturation solubility exceeding 10 g with respect to 100 g of water at 25°C result in use of a large amount of components, and thus it becomes difficult to stabilize the composition for application to skin. For this reason, asparagine, glutamine, histidine, methionine, valine, isoleucine, leucine, phenylalanine, tryptophan, taurine, and the like having a saturation solubility of 1 to 10 g with respect to 100 g of water at 25°C are most suitable for use. When the valuable substance is taurine, the taurine is preferably present in both a dissolved state and a crystalline state in the composition for application to skin.

It is also preferable that the valuable substance is a peptide. Examples of the peptide that can be used in the present invention include dipeptides in which two amino acids are bonded and tripeptides in which three amino acids are bonded. Since there are 20 types of amino acids even limiting to amino acids that constitute proteins, there are dipeptides having a large number, namely 400 or more, of combinations of amino acids. Among them, glycylglycine, carnosine in which β-alanine and histidine are bonded, anserine in which β-alanine and 1-methylhistidine are bonded, valenine in which β-alanine and 3-methylhistidine are bonded, aspartame in which phenylalanine and aspartic acid are bonded, and the like are most suitable for use. There are many types of tripeptides, but glutathione in which glutamic acid, cysteine, and glycine are bound is most suitable for use. Tetrapeptides, which also can be used, are difficult to prepare and, therefore, are not practical.

Furthermore, as the peptide, vegetable proteins such as soybean, corn, and wheat, animal proteins such as milk protein and gelatin, and hydrolyzed peptides prepared by hydrolyzing silk, wool, or the like with an enzyme or an acid can also be used in the present invention.

It is also preferable that the valuable substance is a water-soluble vitamin. Examples of the water-soluble vitamin include vitamin B1 (thiamine), vitamin B2 (riboflavin), vitamin B3 (niacinamide), vitamin B5 (pantothenic acid), vitamin B6 (pyridoxine), vitamin B7 (biotin), vitamin B9 (folic acid), vitamin B12 (cyanocobalamin), and vitamin C.

In addition, derivatives such as thiamine monophosphate, thiamine diphosphate, thiamine triphosphate, flavin mononucleotide, flavin adenine dinucleotide, riboflavin phosphate, nicotinic acid adenine dinucleotide phosphate, pyridoxine cyclic phosphate, hydroxycobalamin, deoxyadenosylcobalamin, ascorbyl glucoside, sodium ascorbyl phosphate, and 3-O-ethyl ascorbic acid can also be utilized.

Among such water-soluble vitamins, those having a low saturation solubility of less than 1 g with respect to 100 g of water at 25°C can form crystals only in a small amount, so that they hardly afford a gritty sense of use. In addition, those having a high saturation solubility exceeding 10 g with respect to 100 g of water at 25°C require use of a large amount of components, and thus it becomes difficult to stabilize the composition for application to skin. For this reason, a valuable substance having a saturation solubility in the range of 1 to 10 g with respect to 100 g of water at 25°C is suitable.

The blending amount of the valuable substance in a crystalline state into the composition for application to skin may be an amount of 1.0 to 5.0 times by mass the saturation solubility with respect to the blending amount of water (moisture content) in the composition. When the valuable substance is added at an amount 5 times the saturation solubility, an increased amount of crystals are precipitated, resulting in an enhanced prickly feel. On the other hand, when the valuable substance is added at an amount close to the saturation solubility, crystals may or may not appear due to a temperature variation depending on the seasons. Therefore, the blending amount of the valuable substance in a crystalline state into the composition for application to skin is preferably 1.2 to 5.0 times by mass the saturation solubility, more preferably 1.5 to 3.0 times by mass, and most preferably 1.5 to 2.0 times by mass, with respect to the amount of water (moisture content) in the composition.

The valuable substance in the present invention is preferably in the state of a needlelike crystal. The crystal size of the valuable substance is desirably 5 um or more and 3000 um or less in length. When the length is less than 5 µm, a prickly feel to the skin is excessively weak. On the other hand, when the length exceeds 3000 µm, irritation to the skin is strong at the time of application to the skin, and there is a risk of skin damage. In addition, the valuable substance having such a large size is hard to be dissolved on the skin and is hard to be absorbed.

Furthermore, in the present invention, it is also possible to blend a crystalline component having low solubility in water as it is in the composition for application to skin. Cymen-5-ol and its isomer, thymol, i.e. isopropylmethylphenol, have solubilities in 100 g of water at 25°C of 0.015 g and 0.098 g, respectively. Therefore, even when 0.1% by mass of cymen-5-ol is added to a composition for application to skin containing 50% by mass of water, the object of the present invention can be attained because most of the cymen-5-ol is present in a state of crystals which are not dissolved in water. The blending amount of cymen-5-ol in the composition for application to skin is 0.01 to 0.2% by mass, preferably 0.01 to 0.1% by mass, and more preferably 0.05 to 0.1% by mass.

The medium of the composition for application to skin to which the valuable substance is added may be an oil-based medium, an aqueous medium, or an emulsified medium in which both an oily medium and an aqueous medium are mixed.

The composition for application to skin in the present invention is typically a cosmetic and is used for skin care.

In the cosmetic, as an aqueous phase component, water, water-soluble alcohols, saccharides, anti-inflammatory agents, thickeners, and so on which are usually used for cosmetics, quasi-pharmaceutical products, and the like can be blended, and as desired, a beauty effect component such as a moisturizer, a whitening agent, a keratin softening component, an anti-aging agent, an anti-saccharification component, a blood circulation promoting component, or polyphenols may be blended. In order to stabilize the composition, a metal ion sequestrant, a water-soluble ultraviolet absorber, a powder component, a preservative, an antioxidant, a pH adjuster, and so on may be appropriately blended, as necessary.

The water contained in the cosmetic is not particularly limited, and examples thereof include purified water, ionexchanged water, and tap water.

Examples of a water-soluble alcohol include lower alcohols, polyhydric alcohols, polyhydric alcohol polymers, divalent alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the foregoing.

Examples of the lower alcohols include ethanol, propanol, isopropanol, isobutyl alcohol, and t-butyl alcohol.

Examples of the polyhydric alcohols include dihydric alcohols (for example, dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, and octylene glycol), trihydric alcohols (for example, glycerin and trimethylolpropane), tetrahydric alcohols (for example, diglycerin and pentaerythritol, such as 1,2,6-hexanetriol), pentahydric alcohols (for example, xylitol and triglycerin), hexahydric alcohols (for example, sorbitol and mannitol), polyhydric alcohol polymers (for example, diethylene glycol, dipropylene glycol-triethylene glycol, polypropylene glycol, tetraethylene glycol, diglycerintriglycerin, tetraglycerin, and polyglycerin), divalent alcohol alkyl ethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether), dihydric alcohol alkyl ethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether triethylene glycol monomethyl ether triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether), dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadibate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate), glycerin monoalkyl ethers (for example, xyl alcohol, selachyl alcohol, and batyl alcohol), sugar alcohols (for example, maltotriose, mannitol, sucrose, erythritol, glucose, fructose, starch-decomposed sugar, maltose, and starch sugar hydrogenated alcohol), glysolid, tetrahydrofurfuryl alcohol, POE-tetrahydrofuryl alcohol, POP-butyl ether, POP-POE-butyl-tertripolyoxypropylene glycerin ether, POP-glycerin ether, POP-glycerin ether phosphoric acid, POP-POE-pentaerythritol ether, and polyglycerin.

Examples of monosaccharides include triose (for example, D-glyceryl aldehyde, and dihydroxyacetone); tetrose (for example, D-erythrose, D- erythrulose, D-threose, and erythritol); pentose (for example, L-arabinose, D-xylose, L-lyxose, D-arabinose, D-ribose, D-ribulose, D-xylulose, and L-xylulose); hexose (for example, D-glucose, D-talose, D-psicose, D-galactose, D-fructose, L-galactose, L-mannose, and D-tagatose); heptose (for example, aldoheptose, and heplose); octose (for example, octulose); deoxy sugar (for example, 2-deoxy-D-ribose, 6-deoxy-L- galactose, and 6-deoxy-L-mannose); amino sugar (for example, D-glucosamine, D-galactosamine, sialic acid, aminouronic acid, and muramic acid); and uronic acid (for example, D-glucuronic acid, D-mannuronic acid, L-guluronic acid, D-galacturonic acid, and L-iduronic acid).

Examples of the oligosaccharide include sucrose, guntianose, umbelliferose, lactose, planteose, isolychnoses, α,α-trehalose, raffinose, lychnoses, umbilicin, and stachyose verbascoses.

Examples of the polysaccharide include cellulose, quince seed, starch, galactan, dermatan sulfate, glycogen, gum arabic, heparan sulfate-tragacanth gum, keratan sulfate, chondroitin, xanthan gum, guar gum, dextran, keratosulfate, locust bean gum, and succinoglucan.

Examples of the anti-inflammatory agent include plantderived components, allantoin and derivatives thereof, glycyrrhetinic acid and derivatives thereof, glycyrrhizic acid and salts or derivatives thereof, salicylic acid derivatives, aminocaproic acid, and azulene and derivatives thereof.

Examples of the thickener include gum arabic, carrageenan, gum karaya, gum tragacanth, carob gum, quince seed (marmelo), casein, dextrin, gelatin, sodium pectinate, sodium araginate, methyl cellulose, ethyl cellulose, CMC, hydroxyethyl cellulose, hydroxypropyl cellulose, PVA, PVM, PVP, sodium polyacrylate, carboxyvinyl polymer, locust bean gum, guar gum, tamarint gum, dialkyldimethylammonium sulfate cellulose, xanthan gum, aluminum magnesium silicate, bentonite, hectorite, A1Mg silicate (Veegum), laponite, and silicic anhydride.

Examples of the natural water-soluble polymer include plant-based polymers (for example, gum arabic, gum tragacanth, galactan, guar gum, carob gum, gum karaya, carrageenan, pectin, agar, quince seed (marmelo), algae colloid (brown algae extract), starch (rice, corn, potato, wheat), and glycyrrhizic acid); microorganism-based polymers (for example, xanthan gum, dextran, succinoglycan, and pullulan); and animal-based polymers (for example, collagen, casein, albumin, and gelatine).

Examples of semi-synthetic water-soluble polymers include starch-based polymers (for example, carboxymethyl starch and methyl hydroxypropyl starch), cellulose-based polymers (methyl cellulose, ethyl cellulose, methyl hydroxypropyl cellulose, hydroxyethyl cellulose, cellulose sodium sulfate, hydroxypropyl cellulose, carboxymethyl cellulose, sodium carboxymethyl cellulose, crystalline cellulose, and cellulose powder), and alginic acid-based polymers (for example, sodium alginate and propylene glycol alginate).

Examples of synthetic water-soluble polymers include vinyl base polymers (for example, polyvinyl alcohol, polyvinyl methyl ether, polyvinylpyrrolidone, and carboxyvinyl polymers); polyoxyethylene base polymers (for example, polyethylene glycol 20,000, 40,000, and 60,000); acrylic polymers (for example, sodium polyacrylate, polyethyl acrylate, and polyacrylamide); polyethyleneimine; and cationic polymers.

Examples of the moisturizer include chondroitin sulfate, hyaluronic acid, mucoitin sulfate, charonic acid, atelocollagen, cholesteryl 12-hydroxystearate, sodium lactate, bile salts, DL-pyrrolidone carboxylate, short-chain soluble collagen, diglycerin (EO) PO adduct, chestnut rose extract, yarrow extract, and melilot extract.

Examples of the whitening component include tranexamic acid, ascorbic acid and salts thereof, vitamin C such as ascorbic acid derivatives (sodium ascorbate phosphate ester, magnesium ascorbate phosphate ester, ascorbyl tetra2-hexyldecanoate, 2-O-ethylascorbic acid, 3-O-ethylascorbic acid, ascorbyl glucoside, etc.), arbutin, kojic acid, placenta, ellagic acid, nicotinamide, hydroquinone, linoleic acid and derivatives thereof.

Examples of the keratin softening component include lactic acid, salicylic acid, gluconic acid, glycolic acid, citric acid, malic acid, fruit acid, phytic acid, urea, and sulfur.

Examples of the anti-aging component include hydrolyzed soybean protein, retinoids (retinol and derivatives thereof, retinoic acid, retinal, and so on), kinetin, adenosine, NMN (nicotinamide mononucleotide), AMP (adenosine monophosphate), ADP (adenosine diphosphate), ATP (adenosine triphosphate), ursolic acid, turmeric extract, sphingosine derivatives, and mevalonolactone.

Examples of the anti-saccharification component include plant extracts such as Buddleja axillaris leaf extract, evening primrose oil, fruit or fruit juice of amla or an extract thereof, L-arginine, L-lysine, hydrolyzed casein, hydrolyzable tannin, and carnosine.

Examples of the blood circulation promoting component include components derived from vegetables such as Asian ginseng, Angelica keiskei, mountain arnica, gingko, fennel, Isodonis japonicus, Dutch oak, chamomile, Roman chamomile, Daucus carota sativa, gentian, burdock, rice, Japanese hawthorn, shiitake mushroom, ginger, English hawthorn, juniper, Cnidium officinale Makino, swertia herb, thyme, clove, citrus unshiu, chili pepper, angelica root, peach kernel, spruce, carrot, garlic, butcher's broom, grape, peony, horse chestnut, lemon balm, yuzu, coix, green tea, rosemary, rose hip, citrus unshiu, angelica, spruce, peach, apricot, walnut, corn, golden chamomile, ichthammol, cantharides tincture, and cepharanthine; gamma oryzanol, tocopherol nicotinate, and glucosyl hesperidin.

Examples of the polyphenols include flavonoid-based polyphenols such as curcuminoids, flavanones, stilpenoids, polymethoxyflavonoids, flavonols, xanthonoids, chalcones, lignoids, flavanols, and isoflavones.

Examples of a metal ion sequestrant include 1-hydroxyethane-1,1-diphosphonic acid, tetrasodium 1-hydroxyethane-1,1-diphosphonate, disodium edetate, trisodium edetate, tetrasorium edetate, sodium citrate, sodium polyphosphate, sodium metaphosphate, gluconic acid, phosphoric acid, citric acid, ascorbic acid, succinic acid, edetic acid, and trisodium hydroxyethyl ethylenediamine triacetate.

Examples of the water-soluble ultraviolet absorbers include benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate salts, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; benzimidazole-based ultraviolet absorbers such as phenylbenzimidazole-5-sulfonic acid and salts thereof, phenylene-bis-benzimidazole-tetrasulfonic acid and salts thereof; 3-(4'-methylbenzylidene)-d,1-camphor, 3-benzylidene-d,1-camphor, urocanic acid, and ethyl urocanate.

Examples of the powder components include inorganic powders (for example, talc, kaolin, mica, sericite, muscovite, phlogopite, synthetic mica, red mica, biotite, permiculite, magnesium carbonate, calcium carbonate, aluminum silicate, barium silicate, calcium silicate, magnesium silicate, strontium silicate, metal tungstates, magnesium, silica, zeolite, barium sulfate, calcined calcium sulfate (calcined gypsum), calcium phosphate, fluorapatite, hydroxyapatite, ceramic powder, metal soap (for example, zinc myristate, and calcium palmitate, aluminum stearate), and boron nitride); organic powders (for example, polyamide resin powder (nylon powder), polyethylene powder, polymethyl methacrylate powder, polystyrene powder, copolymer resin powder of styrene and acrylic acid, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and loess); inorganic black pigments (for example, black iron oxide and low valence titanium oxides); inorganic violet pigments (for example, mango violet and cobalt violet); inorganic green pigments (for example, chromium oxide, chromium hydroxide, and cobalt titanate); inorganic blue pigments (for example, ultramarine blue and iron blue) ; pearl pigments (for example, titanium oxide coated mica, titanium oxide coated bismuth oxychloride, titanium oxide coated talc, colored titanium oxide coated mica, bismuth oxychloride, and argentine); metal powder pigments (for example, aluminum powder, copper powder, or the like); organic pigments such as zirconium, barium or aluminum lake (for example, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1); and natural pigments (for example, chlorophyll and β-carotene).

Examples of the vitamins include vitamins A, B1, B2, B6, C, and E and derivatives thereof, pantothenic acid and derivatives thereof, and biotin.

Examples of antioxidants include tocopherols, dibutylhydroxytoluene, butylhydroxyanisole, and gallate esters.

Examples of the pH adjusters include buffers such as lactic acid-sodium lactate, citric acid-sodium citrate, and succinic acid-sodium succinate.

Examples of the oily medium (oil phase component) to be used for the present invention include liquid oils, solid fats, waxes, hydrocarbon oils, higher fatty acids, synthetic ester oils, and silicone oils which are commonly used in cosmetics and quasi-pharmaceutical products. Such an oil phase, the aqueous phase described above and an appropriate surfactant may be combined and emulsified to afford a composition for application to skin.

Examples of the liquid oils include avocado oil, camellia oil, turtle oil, macadamia nut oil, corn oil, mink oil, olive oil, rapeseed oil, sesame oil, persic oil, wheat germ oil, camellia kissi seed oil, castor oil, linseed oil, safflower oil, cotton seed oil, perilla oil, soybean oil, peanut oil, tea seed oil, rice bran oil, Japanese tung oil, jojoba oil, germ oil, and triglycerol.

Examples of the solid fats include cocoa butter, coconut oil, horse fat, hardened coconut oil, palm oil, hardened beef tallow, palm kernel oil, lard, Japan wax kernel oil, hardened oil, Japan wax, and hardened castor oil.

Examples of the waxes include beeswax, candelilla wax, cotton wax, carnauba wax, bayberry wax, insect wax, montan wax, rice bran wax, lanolin, lanolin acetate, liquid lanolin, corn wax, lanolin fatty acid isopropyl, hexyl laurate, hydrogenated lanolin, jojoba wax, hard lanolin, shellac wax, polyoxyethylene lanolin alcohol ether, polyoxyethylene lanolin alcohol acetate, polyoxyethylene cholesterol ether, polyethyleneglycol lanolin, polyoxyethylene hydrogenated lanolin alcohol ether, and cetyl palmitate.

Examples of the hydrocarbon oils include liquid paraffin, ozokerite, squalane, pristane, paraffin, ceresin. squalene, petrolatum, and microcrystalline wax.

Examples of the higher fatty acid include lauric acid, myristic acid, palmitic acid, stearic acid, behenic acid, oleic acid, undecylenic acid, tall oil acid, linoleic acid, linolenic acid, eicosapentaenoic acid (EPA), and docosahexaenoic acid (DHA).

Examples of the synthetic ester oils include cetyl octanoate, myristyl myristate, glyceryl tri(2-ethylhexanoate), pentaerythritol tetra(2-ethylhexanoate), dioctyl succinate, and tripropylene glycol dineopentanoate.

Examples of the silicone oils include chain polysiloxanes (for example, dimethylpolysiloxane, methylphenylpolysiloxane, and diphenylpolysiloxane); cyclic polysiloxanes (for example, octamethylcyclotetrasiloxane, decamethylcyclopentasiloxane, and docecamethylcyclohexasiloxane); silicon resins with three-dimensional network; silicon rubbers; modified polysiloxanes (for example, amino-modified polysiloxane, polyethermodified polysiloxane, alkyl-modified polysiloxane, and fluorine-modified polysiloxane); and acrylic silicones.

The composition for application to skin of the present invention may also be in a milky to creamy form as a skin care product, and a composition having a viscosity within a range of 2 to 300 Pa.s at 25°C is preferable.

Furthermore, the composition for application to skin of the present invention can also be prepared by gelling an oil such as the hydrocarbon oil, higher fatty acid, synthetic ester oil, or silicone oil with an appropriate oil gelator, and adding the crystallized product produced. As an oil gelator, an appropriate amount of PIONIER GEL 12 PAO (manufactured by Hansen & Rosenthal KG (Germany): available from Matsumoto Trading Co., Ltd.), which is a mixture of a hydrogenated (styrene/isoprene) copolymer and hydrogenated polydecene, fatty acid dextrin (Rheopearl KL2: palmitic acid dextrin), or EMALEX AN-GEL (manufactured by Nihon Emulsion Co., Ltd.), which is an amino acid based gelator is added to and dissolved in an oil, and thus a gel can be prepared.

The method for producing a composition for application to skin of the present invention comprises a step of preparing a solution of a valuable substance at a supersaturation concentration, adding the solution to a composition for application to skin, and precipitating crystals while stirring under cooling. In this step, the solution having a supersaturation concentration may be heated to room temperature or higher during the preparation thereof. After the preparation, the solution is cooled to a temperature equal to or lower than the temperature during the preparation, so that crystals of the valuable substance are produced. The heating and cooling temperatures can be appropriately set according to the valuable substances to be used.

Another method for producing the composition for application to skin of the present invention comprises a dispersion step of preparing part of a valuable substance as a solution having a supersaturation concentration, adding the solution to a composition for application to skin, and precipitating crystals while stirring under cooling; and a step of adding crystals of part of the valuable substance direct to the composition for application to skin and dispersing the part of the valuable substance.

Still another method for producing the composition for application to skin of the present invention comprises a step of adding and dispersing crystals of a valuable substance in a composition for application to skin.

### EXAMPLES

Hereinafter, the present invention will be described in detail with reference to specific examples, but the present invention is not limited to the following Examples. The blending amounts in the following Examples and Comparative Examples are in % by mass unless otherwise specified.

### (Example 1)

An example of a composition for application to skin using taurine as a crystalline component is described. Since the saturation solubility of taurine is 7.9 g/100 g, taurine in an amount 2.38 times the saturation solubility is dissolved in the composition.

The formulation of the composition for application to skin is as follows.
[A]

| | |
|---|---|
| Dimethicone | 29.60% by mass |
| (Dimethicone/vinyl dimethicone) crosspolymer | 3.75% by mass |
| (Dimethicone/(PEG-10/15)) crosspolymer | 2.50% by mass |
| PEG-9 polydimethylsiloxyethyl dimethicone | 2.00% by mass |

[B]

| | |
|---|---|
| Propanediol | 5.00% by mass |
| Phenoxyethanol | 0.30% by mass |
| Taurine | 9.00% by mass |
| Purified water | 47.85% by mass |

The mixture was heated to 80°C, and the mixed phase A was stirred with a homomixer. When the mixed solution of the phase B is heated to 80°C, taurine is completely dissolved to form a transparent solution. The present solution B was added to the phase A over 5 minutes, stirred with a homomixer for 10 minutes, and then cooled to 25°C, affording a composition for application to skin. When the present composition was made into a thin film and microscopically observed, many needlelike crystals of taurine having a length of 500 to 1,000 um were observed.

### (Comparative Example 1)

A composition for application to skin was prepared by changing the blending amount of taurine in Example 1 to 3.3% by mass and the amount of water to 46.7% by mass, adjusting the difference with dimethicone, and setting the other components to have the blending amounts unchanged. The solubility of taurine with respect to 100 g of water at that time was 7.07 g, which was 0.895 times the saturation solubility. When the present composition was made into a thin film and microscopically observed, there were no needlelike crystals of taurine.

### (Comparative Example 2)

A composition for application to skin was prepared by changing the blending amount of taurine in Example 1 to 14.5% by mass and the amount of water to 35.5% by mass, adjusting the difference with dimethicone, and setting the other components to have the blending amounts unchanged. The solubility of taurine with respect to 100 g of water at that time was 40.8 g, which was 5.16 times the saturation solubility. When the present composition was made into a thin film and microscopically observed, it was found that many needlelike crystals of taurine were dispersed and there were also needlelike crystals of 3000 um or more.

### (Comparative Example 3)

A composition for application to skin of Comparative Example 3 was prepared by changing the taurine in Example 1 to 0.1% by mass of hydrolyzed sponge and 49.9% by mass of purified water, adjusting the difference with dimethicone, and setting the other components to have the blending amounts unchanged. The hydrolyzed sponge (Spongilla Spicule Powder ST-A002: manufactured by Toa Kasei Co., Ltd.) is a natural fine needle made of silicon/calcium carbonate having a size of 180 um to 250 um in length and 10 um to 25 um in diameter, and is a component insoluble in water.

### (Example 2)

A composition for application to skin was prepared by changing the blending amount of taurine in Example 1 to 9.6% by mass and the amount of water to 40.4% by mass, adjusting the difference with dimethicone, and setting the other components to have the blending amounts unchanged. The solubility of taurine with respect to 100 g of water at that time was 23.8 g, which was 3.01 times the saturation solubility. When the present composition was made into a thin film and microscopically observed, taurine crystals were scattered, and the length of the crystals was 50 to 1000 µm.

### (Example 3)

A composition for application to skin was prepared by changing the blending amount of taurine in Example 1 to 13.1% by mass and the amount of water to 36.9% by mass, adjusting the difference with dimethicone, and setting the other components to have the blending amounts unchanged. The solubility of taurine with respect to 100 g of water at that time was 35.5 g, which was 4.5 times the saturation solubility. When the present composition was made into a thin film and microscopically observed, needlelike crystals of taurine were observed and the size of the crystals were 50 to 1500 um were observed.

### (Example 4)

An example of a composition for application to skin using pyridoxine hydrochloride, which is a water-soluble vitamin, as a crystalline component is described. Since the saturation solubility of pyridoxine hydrochloride is 22.0 g/100 g, the amount of pyridoxine hydrochloride is 1.5 times the saturation solubility. The formulation of the composition for application to skin is as follows.
[A]

| | |
|---|---|
| Petrolatum | 38.88% by mass |
| Candelilla wax | 1.00% by mass |
| Paraffin | 0.10% by mass |
| DL-α-Tocopherol acetate | 0.10% by mass |
| Hydrogenated lecithin | 1.00% by mass |
| Ethylhexylglycerin | 0.05% by mass |
| Glyceryl caprylate | 0.05% by mass |

[B]

| | |
|---|---|
| Propanediol | 7.50% by mass |
| (Acrylates/alkyl (C10-30) acrylate) crosspolymer | 0.35% by mass |
| Phenoxyethanol | 0.50% by mass |
| Dipotassium glycyrrhizinate | 0.05% by mass |
| Pyridoxine hydrochloride | 12.40% by mass |
| Purified water | 37.60% by mass |

[C]

| | |
|---|---|
| Arginine | 0.42% by mass |

The phase A and the phase B was heated to a temperature of 80 to 85°C, the phase A was then added to the phase B, and the mixture was stirred with a homomixer to be emulsified. After completion of this treatment, the phase C was mixed and cooled to 25°C, affording a composition for application to skin.

### (Example 5)

An example of a composition for application to skin using glycylglycine, which is a dipeptide, as a crystalline component is described. Since the saturation solubility of glycylglycine is 13.2 g/100 g, glycylglycine in an amount 2.5 times the saturation solubility was dissolved. The formulation of the composition for application to skin is as follows.
[A]

| | |
|---|---|
| Isopropyl myristate | 33.70% by mass |
| Jojoba oil | 5.00% by mass |
| Polyglyceryl polyricinoleate-6 | 3.25% by mass |
| Polyglyceryl isostearate-2 | 1.00% by mass |
| Disteardimonium hectorite | 0.75% by mass |

[B]

| | |
|---|---|
| Propanediol | 5.00% by mass |
| Phenoxyethanol | 0.30% by mass |
| Glycylglycine | 12.4% by mass |
| Purified water | 37.6% by mass |

The mixture was heated to 80°C, and while the mixed phase A was stirred with a homomixer, the mixed solution of the phase B heated to 80°C was added over 5 minutes. The mixture was stirred with a homomixer for 10 minutes, and then cooled to 25°C, affording a composition for application to skin. When the present composition was made into a thin film and microscopically observed, many crystals equal to or shorter than 1000 um were observed.

### (Example 6)

An example of a composition for application to skin using glutathione, which is a tripeptide, as a crystalline component is described. Since the saturation solubility of glutathione is 13.0 g/100 g, glutathione in an amount 2.0 times the saturation solubility was dissolved. The formulation of the composition for application to skin is as follows.
[A]

| | |
|---|---|
| Dimethicone | 40.10% by mass |
| Tocopherol | 0.10% by mass |
| PEG-10 dimethicone | 1.75% by mass |
| Lauryl PEG-9 polydimethylsiloxyethyl dimethicone | 0.75% by mass |
| Disteardimonium hectorite | 1.00% by mass |

[B]

| | |
|---|---|
| Propanediol | 5.00% by mass |
| Phenoxyethanol | 0.30% by mass |
| Glutathione | 10.5% by mass |
| Purified water | 40.5% by mass |

The mixture was heated to 80°C, and while the mixed phase A was stirred with a homomixer, the mixed solution of the phase B heated to 80°C was added over 5 minutes. The mixture was stirred with a homomixer for 10 minutes, and then cooled to 25°C, affording a composition for application to skin.

### (Example 7)

An example of a non-aqueous composition for application to skin using tryptophan, which is an amino acid, as a crystalline component is described. In 10 g of water was dissolved 2.12 g of tryptophan, and the solution was left at 5°C for a whole day and night to precipitate crystals. After removing moisture and drying, crystals were obtained. The crystals obtained were blended in the following formulation, affording a non-aqueous composition for application to skin.
[A]

| | |
|---|---|
| Cetyl ethylhexanoate | 62.40% by mass |
| Hydrogenated polydecene | 29.00% by mass |
| Hydrogenated (styrene/isoprene) copolymer | 7.00% by mass |
| Tocopherol | 0.10% by mass |

[B]

| | |
|---|---|
| Tryptophan crystal | 1.50% by mass |

The phase A was mixed, and heated to 90°C with stirring to uniformly dissolve the ingredients. The phase B was added, and the mixture was stirred for 10 minutes and then cooled to 25°C, affording a composition for application to skin.

### (Example 8)

An example of a composition for application to skin using taurine and cymen-5-ol as crystalline components is described. Since the saturation solubility of taurine is 7.9 g/100 g, taurine in an amount 1.80 times the saturation solubility was dissolved. The formulation of the composition for application to skin is as follows.
[A]

| | |
|---|---|
| Petrolatum | 40.80% by mass |
| Candelilla wax | 1.00% by mass |
| DL-α-Tocopherol acetate | 0.10% by mass |
| Hydrogenated lecithin | 1.00% by mass |
| Ethylhexylglycerin | 0.05% by mass |
| Glyceryl caprylate | 0.05% by mass |

[B]

| | |
|---|---|
| Propanediol | 7.50% by mass |
| (Acrylates/alkyl (C10-30) acrylate) crosspolymer | 0.35% by mass |
| Phenoxyethanol | 0.50% by mass |
| Dipotassium glycyrrhizinate | 0.05% by mass |
| Taurine | 6.00% by mass |
| Cymen-5-ol | 0.10% by mass |
| Purified water | 38.30% by mass |

[C]

| | |
|---|---|
| Arginine | 0.42% by mass |
| Purified water | 3.78% by mass |

The phase A and the phase B was heated to a temperature of 80 to 85°C, the phase A was then added to the phase B, and the mixture was stirred with a homomixer to be emulsified. After completion of this treatment, the phase C was mixed and cooled to 25°C, affording a composition for application to skin.

### (Example 9) Evaluation of composition for application to skin

For the compositions for application to skin prepared in Examples, the sense of use was evaluated by specialized panelists. Five panelists evaluated the sense of use and the sense of irritation according to the following evaluation items.

| Evaluation of sense of use | |
|---|---|
| A prickly feel was felt very strongly. | 5 |
| A prickly feel was strongly felt. | 4 |
| A prickly feel was felt. | 3 |
| A prickly feel was felt a little. | 2 |
| A prickly feel was slightly felt. | 1 |
| No prickly feel was felt. | 0 |

### Evaluation of sense of irritation

A strong sense of irritation remained after application.
5

A sense of irritation remained after application.
3

A slight sense of irritation remained after application.
1

No sense of irritation was felt after application. 0

**[Table 1]**

| | Example 1 | Example 2 | Example 3 | Example 4 | Example 5 | Example 6 | Example 7 | Example 8 |
|---|---|---|---|---|---|---|---|---|
| Rating of sense of use | 21 | 23 | 24 | 22 | 22 | 21 | 20 | 24 |
| Rating of sense of irritation | 1 | 1 | 5 | 1 | 2 | 0 | 1 | 2 |

**[Table 2]**

| | Comparative Example 1 | Comparative Example 2 | Comparative Example 3 |
|---|---|---|---|
| Rating of sense of use | 0 | 21 | 25 |
| Rating of sense of irritation | 0 | 17 | 25 |

Table 1 shows sensory evaluation of eight compositions for application to skins of Examples of the present invention. In all the cases, a prickly feel due to the crystal component was felt, but after the composition was blended with the skin, a sense of irritation was not felt any longer due to dissolution of the crystal component.

Table 2 shows sensory evaluation of three compositions for application to skins of Comparative Examples. The composition for application to skin of Comparative Example 1 was not crystallized and a prickly feel was not felt because of the blending with a blending concentration of taurine being a concentration equal to or lower than the saturation solubility. In Comparative Example 2, since the crystal component was blended at a concentration of 5 times or more the saturation solubility concentration, the formation of crystals was inhibited, a prickly feel was felt at a low rate, and a gritty feel was felt. In addition, after application to the skin, the component was poorly blended with the skin and the crystal component was not completely dissolved and the crystal component rubbed the skin in a crystalline state, so that a strong sense of irritation was felt. Furthermore, in the case where the hydrolyzed sponge was added described in Comparative Example 3, a prickly feel was felt strongly, but due to the substance that was not dissolved, fine needles remained on the skin for a long time and a very strong sense of irritation remained indefinitely.

As described above, it has been clarified that the compositions for application to skin prepared by blending taurine as an amino acid, pyridoxine hydrochloride as a water-soluble vitamin, glycylglycine as a dipeptide, or glutathione as a tripeptide, respectively, in an amount of 1.0 to 5.0 times the saturated solution concentration, and a composition for application to skin prepared by blending cymen-5-ol were all compositions for application to skin having a prickly feel but failing to afford a sense of skin irritation after being blended with the skin.

### Example 10

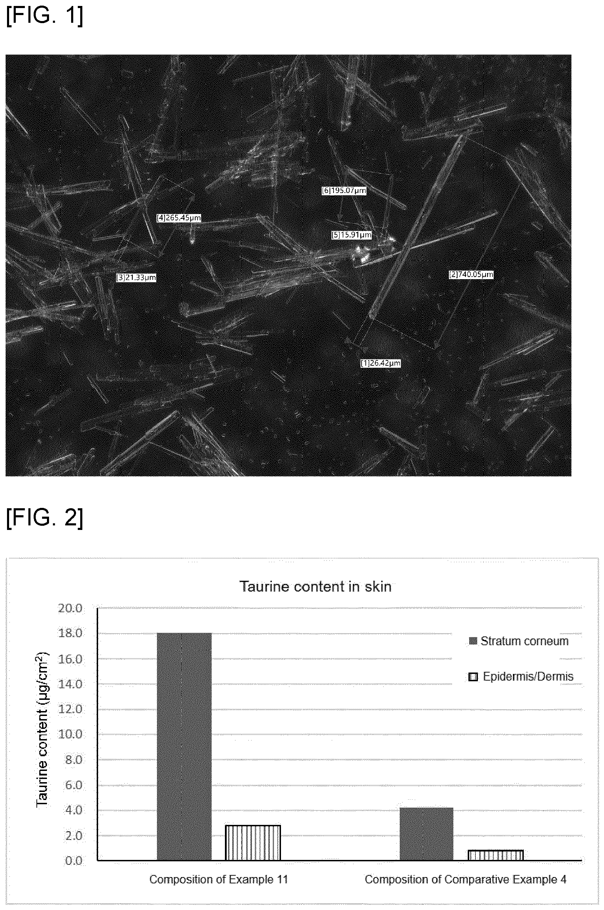

An aqueous solution (100 mL) containing 10% by mass of taurine was heated to 60°C until uniform dissolution, and then this solution was slowly added to 15 mL of ethanol over 5 minutes with vigorously stirring with a disper to precipitate crystals. The precipitated crystals were collected by filtration, washed with ethanol, and then dried, affording taurine crystals. The crystals obtained were confirmed to be needlelike crystals having a maximum thickness of 26.4 micrometers and a maximum length of 740.1 micrometers from microscopic observation as shown in FIG. 1.

### Example 11 and Comparative Example 4

In accordance with the blending amounts given in Table 3, a composition of Example 11 containing 10% by mass of the taurine crystals obtained in Example 10 and a composition of Comparative Example 4 not containing the crystals were prepared. Then, the compositions obtained were applied to human skin and the amounts of taurine permeated into the skin were compared.

**[Table 3]**

| | Example 11 | Comparative Example 4 |
|---|---|---|
| Purified water | 81.6% by mass | 91.6% by mass |
| Taurine | 8.0% by mass | 8.0% by mass |
| Carboxyvinyl polymer | 0.2% by mass | 0.2% by mass |
| Arginine | 0.2% by mass | 0.2% by mass |
| Taurine crystals of Example 10 | 10.0% by mass | - |

In the evaluation of the amount of permeation into the skin, each sample was applied to a human excised skin at 10.0 mg/square centimeter using a vertical Franz diffusion cell having an effective diameter of 2.0 centimeters, an area of 3.14 square centimeters, and a receptor fluid capacity of 2.4 mL. After 6 hours, the stratum corneum and the epidermis/dermis were separated from the sample skin by tape stripping, and dipotassium glycyrrhizinate contained in each of the stratum corneum and the epidermis/dermis was quantified by high performance liquid chromatography.

As shown in FIG. 2, as compared with the composition of Comparative Example 4, the composition of Example 11 had an amount of permeation of taurine into the entire skin was about 4.1 times, and it has been confirmed that the amount of permeation into the keratin, the epidermis and the dermis was superiorly increased by forming needlelike crystals.

Formulation examples are shown below, but the present invention is not limited to these examples.

### [Skin lotion]

[A]

| | |
|---|---|
| Purified water | 71.60% by mass |
| Taurine | 7.80% by mass |
| Glycerin | 5.00% by mass |
| Diglycerin | 0.50% by mass |
| Betaine | 0.50% by mass |
| Phenoxyethanol | 0.30% by mass |

[B]

| | |
|---|---|
| Dipropylene glycol | 10.00% by mass |
| 1,2-Pentanediol [C] | 2.50% by mass |
| (Acrylates/steareth-20 methacrylate) copolymer | 0.45% by mass |
| Purified water | 1.05% by mass |

[D]

| | |
|---|---|
| Crystal obtained in Example 10 | 0.30% by mass |

The phase A heated to 80°C and uniformly dissolved was cooled to 35°C, added to the phase B with stirring, and the resulting mixture was stirred at 35°C for 10 minutes. To the mixture was added the phase C, and the resulting mixture was stirred with a homomixer for 10 minutes to uniformly mix. Thereafter, crystals of the phase D were added thereto, and the resulting mixture was stirred for 5 minutes, affording a skin lotion composition.

The crystals of taurine in the composition had a needlelike shape and had a maximum thickness of 26.4 micrometers and a maximum length of 740.1 micrometers. The composition had a smooth sense of use, and a composition having a slightly prickly feel only at the time of application and having a good sense of use was obtained.

### [Aqueous gel]

[A]

| | |
|---|---|
| Purified water | 71.70% by mass |
| Taurine | 8.0% by mass |
| Glycerin | 5.00% by mass |
| Raffinose | 0.50% by mass |

[B]

| | |
|---|---|
| Dipropylene glycol | 10.00% by mass |
| 1,2-Pentanediol | 2.50% by mass |

[C]

| | | |
|---|---|---|
| Polyacrylate crosspolymer-6 | 2.00% by mass | |

[D]

| | |
|---|---|
| Phenoxyethanol | 0.30% by mass |

The phase A heated to 80°C and uniformly dissolved was cooled to 60°C, added to the phase B with stirring, and the resulting mixture was stirred at 60°C for 10 minutes to precipitate taurine crystals. To the mixture was added the phase C, and the resulting mixture was stirred with a homomixer for 10 minutes to uniformly mix. Then, the mixture was cooled to 35°C, and the phase D was added. Then, the resulting mixture was stirred for 5 minutes, affording an aqueous gel composition.

The crystals of taurine in the composition had a needlelike shape and had a maximum thickness of 38.1 micrometers and a maximum length of 683.4 micrometers. The composition obtained had a smooth sense of use, and a composition having a slightly prickly feel only at the time of application and having a good sense of use was obtained.

### [Milky lotion]

[A]

| | |
|---|---|
| Purified water | 61.10% by mass |
| Taurine | 8.00% by mass |
| Glycerin | 5.00% by mass |

[B]

| | |
|---|---|
| 1,3-Propanediol | 15.00% by mass |
| Tranexamic acid | 2.00% by mass |
| Dipotassium glycyrrhizinate | 0.10% by mass |
| Pentylene glycol | 1.50% by mass |
| Phenoxyethanol | 0.30% by mass |

[C]

| (Hydroxyethyl acrylate/Na acryloyldimethyltaurate) | |
|---|---|
| copolymer | 0.75% by mass |
| Isohexadecane | 0.45% by mass |
| Polysorbate 80 | 0.15% by mass |
| Purified water | 0.65% by mass |

[D]

| | |
|---|---|
| Squalane | 5.00% by mass |

The phase A heated to 80°C and uniformly dissolved was cooled to 35°C, added to the phase B with stirring, and the resulting mixture was stirred at 35°C for 10 minutes to precipitate taurine crystals. To this was added the mixture of the phase C, and the resultant was stirred with a homomixer for 5 minutes to uniformly mix. Then, the phase D was added, and the resulting mixture was stirred with a homomixer for 10 minutes to emulsify, affording a milky lotion composition.

The crystals of taurine in the composition had a needlelike shape and had a maximum thickness of 28.6 micrometers and a maximum length of 212.7 micrometers. The composition obtained had a smooth sense of use, and a composition having a slightly prickly feel only at the time of application and having a good sense of use was obtained.

### [O/W type cream]

[A]

| | |
|---|---|
| Purified water | 63.00% by mass |
| Taurine | 8.00% by mass |
| Glycerin | 5.00% by mass |
| Nicotinamide | 5.00% by mass |

[B]

| | |
|---|---|
| 1,3-Propanediol | 15.00% by mass |
| Pentylene glycol | 1.50% by mass |
| Phenoxyethanol | 0.30% by mass |

[C]

| (Hydroxyethyl acrylate/Na acryloyldimethyltaurate) | |
|---|---|
| copolymer | 0.75% by mass |
| Polysorbate 80 | 0.15% by mass |
| Purified water | 0.65% by mass |

[D]

| | |
|---|---|
| Squalane | 0.65% by mass |

The phase A heated to 80°C and uniformly dissolved was cooled to 35°C, added to the phase B with stirring, and the resulting mixture was stirred at 35°C for 10 minutes to precipitate taurine crystals. To this was added the mixture of the phase C, and the resultant was stirred with a homomixer for 5 minutes to uniformly mix. Then, the phase D was added, and the resulting mixture was stirred with a homomixer for 10 minutes to emulsify, affording an O/W type cream composition.

The crystals of taurine in the composition had a needlelike shape and had a maximum thickness of 40.2 micrometers and a maximum length of 824.0 micrometers. The composition obtained had a smooth sense of use, and a composition having a slightly prickly feel only at the time of application and having a good sense of use was obtained.

### [W/O type cream]

[A]

| | |
|---|---|
| Purified water | 32.30% by mass |
| Taurine | 7.00% by mass |
| Glycerin | 5.00% by mass |
| L-Ascorbic acid 2-glucoside | 2.00% by mass |

[B]

| | |
|---|---|
| 1,3-Propanediol | 9.00% by mass |
| 1,2-Hexanediol | 0.70% by mass |
| Phenoxyethanol | 0.30% by mass |

[C]

| | |
|---|---|
| Isopropyl myristate | 33.70% by mass |
| Jojoba oil | 5.00% by mass |
| Polyglyceryl polyricinoleate-6 | 3.25% by mass |
| Polyglyceryl isostearate-2 | 1.00% by mass |
| Disteardimonium hectorite | 0.75% by mass |

The phase A heated to 80°C and uniformly dissolved was cooled to 35°C, added to the phase B with stirring, and the resulting mixture was stirred at 35°C for 10 minutes to precipitate taurine crystals. This mixed liquid was added to the homogeneously mixed phase C over 5 minutes while applying a homomixer, and then stirred and emulsified with the homomixer for 10 minutes, affording a W/O type cream composition.

The crystals of taurine in the composition had a needlelike shape and had a maximum thickness of 35.8 micrometers and a maximum length of 653.5 micrometers. The composition obtained had a smooth sense of use, and a composition having a slightly prickly feel only at the time of application and having a good sense of use was obtained.

### [Cosmetic oil]

[A]

| | |
|---|---|
| Cetyl 2-ethylhexanoate | 60.40% by mass |
| Hydrogenated polydecene | 29.00% by mass |
| Hydrogenated (styrene/isoprene) copolymer | 7.00% by mass |
| Ascorbyl tetrahexyldecanoate | 3.00% by mass |
| Tocopherol | 0.10% by mass |

[B]

| | |
|---|---|
| Taurine crystal obtained in Example 10 | 0.50% by mass |

The phase A was heated to 100°C and stirred until becoming uniform. The mixture was cooled to 35°C, the B phase was added thereto, and the resulting mixture was stirred to uniformly disperse, affording a cosmetic oil composition.

The crystals of taurine in the composition had a needlelike shape and had a maximum thickness of 26.4 micrometers and a maximum length of 740.1 micrometers. The composition obtained had a smooth sense of use, and a composition having a slightly prickly feel only at the time of application and having a good sense of use was obtained.

## Claims

1. A skin care composition for application to skin, comprising a valuable substance that is dispersed in a crystalline state.

2. A skin care composition for application to skin, comprising a valuable substance that is dispersed in a crystalline state, wherein the valuable substance is a needlelike crystal, and a length of the crystal is 5 um or more and 3000 um or less.

3. The skin care composition for application to skin according to claim 1 or 2, wherein the valuable substance is water-soluble.

4. The skin care composition for application to skin according to any one of claims 1 to 3, wherein the valuable substance is an amino acid.

5. The skin care composition for application to skin according to any one of claims 1 to 4, wherein the valuable substance is taurine.

6. The skin care composition for application to skin according to any one of claims 1 to 5, wherein the valuable substance is taurine, and is present in both a dissolved state and a crystalline state.

7. The skin care composition for application to skin according to any one of claims 1 to 3, wherein the valuable substance is a peptide.

8. The skin care composition for application to skin according to any one of claims 1 to 3, wherein the valuable substance is a water-soluble vitamin.

9. The skin care composition for application to skin according to any one of claims 1 to 8, wherein the blending amount of the valuable substance in the composition is an amount of 1.0 to 5.0 times the saturation solubility with respect to the moisture content in the composition.

10. The skin care composition for application to skin according to any one of claims 1 to 9, wherein a medium of the composition for application to skin is an oily medium, an aqueous medium, or an emulsified medium in which an oily medium and an aqueous medium are mixed.

11. The skin care composition for application to skin according to any one of claims 1 to 3, wherein the valuable substance dispersed in a crystalline state is cymen-5-ol or thymol.

12. A method for producing a skin care composition for application to skin, the method comprising a step of preparing a solution of a valuable substance at a supersaturation concentration, adding the solution to a composition for application to skin, and precipitating crystals while stirring under cooling.

13. A method for producing a skin care composition for application to skin, the method comprising a dispersion step of preparing part of a valuable substance as a solution having a supersaturation concentration, adding the solution to a composition for application to skin, and precipitating crystals while stirring under cooling; and a step of adding part of the valuable substance direct as crystals to the composition for application to skin and dispersing the part of the valuable substance.

14. A method for producing a skin care composition for application to skin, the method comprising a step of adding crystals of a valuable substance to a composition for application to skin and dispersing the crystals.
